(19) **Europäisches Patentamt**

**European Patent Office**

**Office européen des brevets**

(11) **EP 1 736 102 A2**

(12) **EUROPEAN PATENT APPLICATION**

(43) Date of publication:
**27.12.2006 Bulletin 2006/52**

(51) Int Cl.:
**A61B 6/03** (2006.01)

(21) Application number: **06253233.8**

(22) Date of filing: **22.06.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **24.06.2005 JP 2005184720**

(71) Applicant: **GE Medical Systems Global Technology Company, LLC Waukesha, Wisconsin 53188-1696 (US)**

(72) Inventors:
• **Nishide, Akihiko**
  **Hino-shi,Tokyo 191-8503 (JP)**
• **Imai, Yasuhiro**
  **Hino-shi,Tokyo 191-8503 (JP)**

(74) Representative: **Pedder, James Cuthbert London Patent Operation, General Electric International, Inc., 15 John Adam Street London WC2N 6LU (GB)**

(54) **X-Ray ct method and x-ray ct apparatus**

(57)    In an X-ray CT apparatus including an X-ray area detector that has a matrix structure and is represented by a multi-array X-ray detector or a flat-panel X-ray detector, radiographic conditions (protocols) for imaging of each position represented by a z-coordinate are optimized in relation to an X-ray cone beam that spreads in a z direction. A slice thickness of a tomographic image is freely controlled in the z direction using a z filter during a conventional scan or a cine scan. For each tomographic image, a reconstruction function, an image filter, a scan field, a tomographic-image tilt angle, and a position of a tomographic image are freely adjusted or independently designated for scanning of each position in the z direction. Thus, a tomographic image is reconstructed. Moreover, the position of a beam formation X-ray filter in the z direction is shifted in order to optimize a patient dose that depends on the size of the scan field, and X-ray quality.

Fig. 2

**Description**

The present invention relates to an X-ray CT method and an X-ray CT apparatus in which radiographic conditions for a conventional scan or a cine scan are designated.

In X-ray CT apparatuses including an X-ray area detector represented by a multi-array X-ray detector or a flat-panel X-ray detector, only one set of radiographic conditions (protocols) is designated for a conventional scan (which may be called an axial scan) for which X-rays are rotationally irradiated once or a cine scan that holds a subject immobile at one scanned position. Moreover, freedom in designating the radiographic condition in relation to one rotational radiation of an X-ray cone beam is limited. This poses a problem in terms of optimization of the radiographic condition.

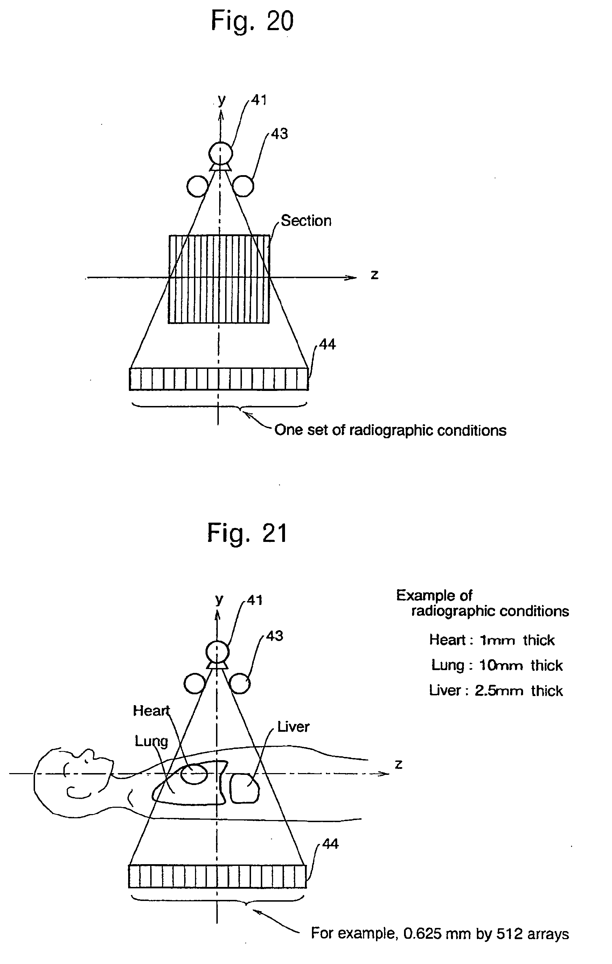

Fig. 20 shows an X-ray data acquisition assembly included in a conventional X-ray CT apparatus. The X-ray data acquisition assembly includes an X-ray tube 41 that generates X-rays, a collimator 43 that controls an X-ray field, and a multi-array X-ray detector 44 that detects X-rays. Subject's sections whose tomographic images are reconstructed are radiographed under the same set of radiographic conditions.

Moreover, X-ray CT apparatuses in which an X-ray beam is divided into portions and the X-ray beam portions are used to reconstruct respective tomographic images or the entire X-ray beam is used to reconstruct a tomographic image are known (refer to, for example, Patent Document 1).

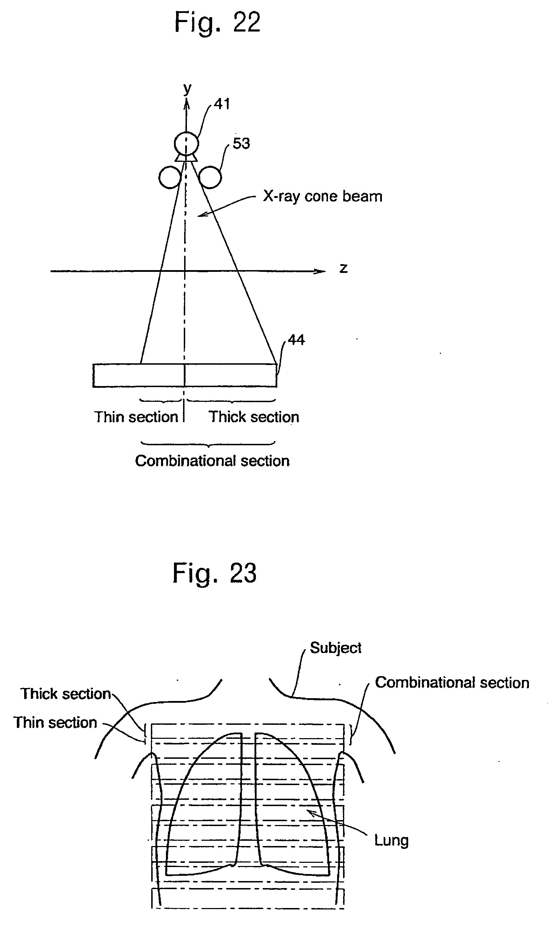

Fig. 22 shows an X-ray data acquisition assembly included in the above X-ray CT apparatus. The X-ray data acquisition assembly includes an X-ray tube 41 that generates X-rays, a collimator 53 that controls an X-ray field, and a multi-array X-ray detector 44 that detects X-rays. The collimator 53 divides an X-ray beam into portions that are asymmetric in a z direction that is a thickness direction. Thus, an image of a thin section, a thick section, or a combinational section of them is reconstructed.

Fig. 23 shows sections of the lungs to be imaged by the X-ray CT apparatus. After a tomographic image of a thick section out of the sections of the lungs shown in Fig. 23 or a tomographic image of a combinational section is used to perform screening, if a tomographic image of a thin section is used to perform a close examination, it is known to be clinically effective. In this case, the thickness of the thick section or combinational section is usually approximately 10 mm, and the thickness of the thin section is usually approximately 1 mm.

For example, assuming that a subject is examined for lung cancer, a multi-slice scan is performed to image relatively thick slices so that the subject's lungs can be radiographed efficiently unintermittently by scanning a limited number of slices. In addition, a multi-slice scan is performed to scan thin slices so that the details of the lungs can be visualized clearly. However, since the number of slices is limited, the multi-slice scan for scanning thin slices is performed on slices separated from one another.

A slice thickness designated as a condition is held intact during production of a series of tomographic images. When the multi-slice scan is performed, the lung fields are entirely radiographed unintermittently by scanning thick slices. Thereafter, the collimator or the like is used to re-designate a slice thickness as a condition, and thin slices are scanned. This is inefficient.

Japanese Unexamined Patent Publication No. 10-305027 (P.1 and Fig. 1) describes a radiation tomography method and system intended to solve the foregoing problem and to produce a plurality of tomographic images of different slice thicknesses during one scan.

In X-ray CT apparatuses including an X-ray area detector represented by a multi-array X-ray detector or a flat-panel X-ray detector, the width in a z direction of the X-ray detector will be presumably as large as 100 mm or 200 mm along with an increase in the conical angle of an X-ray cone beam. This leads to an increase in the width in a z-axis direction of the X-ray cone beam that is rotationally irradiated once for a conventional scan or for a cine scan for which a scanned position is fixed (the z-axis direction corresponds to the direction of an axis of rotation of a data acquisition system composed of an X-ray generator and the X-ray area detector, the body-axis direction of a subject, and a depth direction in which a cradle of a radiographic table moves). The possibility that a plurality of organs may fall within a radiographic range defined with one rotational irradiation of the X-ray cone beam gets higher.

Fig. 21 illustrates a plurality of organs falling within the radiographic range defined with one rotational irradiation of an X-ray cone beam. The heart, lungs, liver, and other organs fall within the radiographic range defined with one rotational reception of the X-ray cone beam by the multi-array X-ray detector 44.

As mentioned above, according to the prior art, one organ may be radiographed with a slice thickness of tomographic images varied depending on a purpose of examination. However, an idea that a plurality of organs is radiographed by diversifying a radiographic method during one irradiation of an X-ray cone beam has not been proposed. In the future, one irradiation of an X-ray beam will have to cope with a plurality of organs and diverse purposes of examination.

Therefore, the present invention seeks to provide an X-ray CT method and an X-ray CT apparatus making it possible to optimize radiographic conditions for a conventional scan or a cine scan of scanning one position in a z direction, which is performed by an X-ray CT apparatus including an X-ray area detector that has a matrix structure and that is represented by a conventional multi-array detector or a flat-panel X-ray detector, in relation to one rotational irradiation of an X-ray cone beam, or making it possible to freely designate the radiographic conditions.

The present invention provides an X-ray CT method or an X-ray CT apparatus making it possible to optimize radiographic conditions or freely designate radiographic conditions in a case where an X-ray cone beam that spreads in a z direction is used to radiograph a wide range extending in the z direction during a conventional scan that requires one rotational irradiation of the X-ray cone beam or during a cine scan that images one position in the z direction.

In efforts to solve the aforesaid problem and accomplish the above object, an X-ray CT method in accordance with the first aspect of the present invention is such that an X-ray generator and an X-ray area detector having a matrix structure and being opposed to the X-ray generator are rotated about a center of rotation located between the X-ray generator and X-ray area detector, X-ray projection data of a subject laying down near the center of rotation is acquired, the acquired X-ray projection data is treated in order to reconstruct an image, a reconstructed tomographic image is displayed, and radiographic conditions for data acquisition are designated. Herein, as the radiographic conditions, a plurality of sets of radiographic conditions is designated for a conventional scan for which the X-ray generator and X-ray area detector make one rotation or for a cine scan for which the position of the subject in a depth direction substantially orthogonal to a rotation plane on which the rotation is made is retained at one position. The image reconstruction is performed under the plurality of sets of radiographic conditions during the conventional scan or cine scan.

In the first aspect of the present invention, a plurality of sets of radiographic conditions is designated for a conventional scan for which the X-ray generator and X-ray area detector make one rotation or for a cine scan for which the position of a subject is fixed in a depth direction substantially orthogonal to a rotation plane on which the rotation is made. During the conventional scan or cine scan, image reconstruction is performed under the plurality of sets of radiographic conditions. Thus, the radiographic conditions are optimized and freedom in designating the radiographic conditions is intensified.

An X-ray CT method in accordance with the second aspect of the present invention is such that an X-ray generator and an X-ray area detector having a matrix structure and being opposed to the X-ray generator are rotated about a center of rotation located between the X-ray generator and the X-ray area detector, X-ray projection data of a subject lying down near the center of rotation is acquired, the acquired X-ray projection data is treated in order to reconstruct an image, a reconstructed tomographic image is displayed, and radiographic conditions for data acquisition are designated. Herein, for data acquisition, an X-ray beam that is asymmetric in a direction orthogonal to a rotation plane on which the rotation is made is irradiated to the rotation plane. For a conventional scan for which the X-ray generator and X-ray area detector make one rotation or a cine scan for which the position of the subject is fixed in a depth direction substantially orthogonal to the rotation plane on which the rotation is made, a plurality of sets of radiographic conditions is designated. During the conventional scan or cine scan, image reconstruction is performed under the plurality of sets of radiographic conditions.

According to the second aspect of the present invention, an X-ray beam is asymmetric to the rotation plane. For a conventional scan for which the X-ray generator and X-ray area detector make one rotation or a cine scan for which the position of the subject is fixed in a depth direction substantially orthogonal to the rotation plane on which the rotation is made, a plurality of sets of radiographic conditions is designated. During the conventional scan or cine scan, image reconstruction is performed under the plurality of sets of radiographic conditions. In relation to the asymmetric X-ray beam, the radiographic conditions are optimized and freedom in designating the radiographic conditions is intensified.

An X-ray CT method in accordance with the third aspect of the present invention is identical to the X-ray CT method in accordance with the second aspect except that a collimator disposed in front of an X-irradiation port of the X-ray generator has an asymmetric structure.

According to the third aspect of the present invention, even when the X-ray collimator is asymmetrical in a x direction, if a radiographic condition designating means capable of designating a plurality of sets of radiographic conditions for a conventional (axial) scan for which the X-ray generator and X-ray area detector make one rotation or a cine scan that scans one position in the z direction, and an image reconstruction means are included, the radiographic conditions can be optimized and freedom in designating the radiographic conditions can be intensified.

An X-ray CT method in accordance with the fourth aspect of the present invention is such that an X-ray generator and an X-ray area detector having a matrix structure and being opposed to the X-ray generator are rotated about a center of rotation located between the X-ray generator and X-ray area detector, X-ray projection data of a subject lying down near the center of rotation is acquired, the acquired X-ray projection data is treated in order to reconstruct an image, a reconstructed tomographic image is displayed, and radiographic conditions for data acquisition are designated. Herein, a rotation plane on which the rotation is made is tilted prior to data acquisition. For a conventional scan for which the X-ray generator and X-ray area detector make one rotation or a cine scan for which the position of the subject is fixed in a depth direction substantially orthogonal to the rotation plane on which the rotation is made, a plurality of radiographic conditions is designated. During the conventional scan or cine scan, image reconstruction is performed under the plurality of sets of radiographic conditions.

According to the fourth aspect of the present invention, the rotation plane on which the rotation is made is tilted prior to data acquisition. For a conventional scan for which the X-ray generator and X-ray area detector make one rotation or a cine scan for which the position of the subject is fixed in a depth direction substantially orthogonal to the rotation plane on which the rotation is made, a plurality of sets of radiographic conditions is designated. During the conventional scan or cine scan, image reconstruction is performed under the plurality of sets of radiographic conditions. With the rotation plane tilted, the radiographic conditions are optimized and freedom in designating the radiographic conditions is intensified.

An X-ray CT method in accordance with the fifth aspect of the present invention is such that an X-ray generator and an X-ray area detector having a matrix structure and being opposed to the X-ray generator are rotated about a center of rotation located between the X-ray generator and the X-ray area detector, X-ray projection data of a subject lying down near the center of rotation is acquired, the X-ray projection data acquired by an X-ray data acquisition means is treated for image reconstruction by an image reconstruction means, a reconstructed tomographic image is displayed, radiographic conditions for data acquisition are designated, and radiographic conditions recommended for each radiographic region of the subject are stored. Herein, for a conventional scan for which the X-ray generator and X-ray area detector make one rotation or a cine scan for which the position of the subject is fixed in a depth direction substantially orthogonal to a rotation plane on which the rotation is made, a radiographic region is delineated in a scout image of the subject, radiographic conditions recommended for the delineated radiographic region are retrieved, and a plurality of sets of radiographic conditions is designated based on the recommended radiographic conditions. During the conventional scan or cine scan, image reconstruction is performed under the plurality of sets of radiographic conditions.

According to the fifth aspect of the present invention, for a conventional scan for which the X-ray generator and X-ray area detector make one rotation or a cine scan for which the position of the subject is fixed in a depth direction substantially orthogonal to the rotation plane on which the rotation is made, a radiographic region is delineated in a scout image of the subject, radiographic conditions recommended for the delineated radiographic region are retrieved, and a plurality of sets of radiographic conditions is designated based on the recommended radiographic conditions. During the conventional scan or cine scan, image reconstruction is performed under the plurality of sets of radiographic conditions. By delineating a radiographic region in a scout image of a subject, radiographic conditions recommended for each radiographic region are automatically designated.

An X-ray CT method in accordance with the sixth aspect of the present invention is identical to the X-ray CT method in accordance with any of the first to fifth aspects except that the X-ray area detector is a multi-array X-ray detector or a flat-panel X-ray detector.

According to the sixth aspect of the present invention, the two-dimensional X-ray area detector may be either a multi-array X-ray detector or a flat-panel X-ray detector. Whether an X-ray beam is asymmetric or symmetric in a z direction, if a radiographic condition designation means to be used to designate a plurality of sets of radiographic conditions for a conventional scan for which the X-ray generator and X-ray area detector make one rotation or a cine scan of scanning one position in the z direction, and an image reconstruction means are included, the radiographic conditions can be optimized and freedom in designating the radiographic conditions can be intensified.

An X-ray CT method in accordance with the seventh aspect of the present invention is identical to the X-ray CT method in accordance with any of the first to sixth aspects except that the radiographic conditions include at least one of a slice thickness, a scan field, a reconstruction function, an image filter, a tomographic-image tilt angle, a position of a tomographic image, and an inter-tomographic image spacing.

According to the seventh aspect of the present invention, if the radiographic condition designation means can be used to designate a slice thickness, a scan field, an image filter, a tomographic-image tilt angle, and a position of a tomographic image, radiographic conditions can be optimized or designated freely.

An X-ray CT method in accordance with the eighth aspect of the present invention is identical to the X-ray CT method in accordance with any of the first to seventh aspects except that image reconstruction is three-dimensional image reconstruction.

According to the eighth aspect of the present invention, a z filter is used to freely vary a slice thickness, and a tomographic-image tilt angle and a position of a tomographic image are freely varied. Thus, radiographic conditions are optimized or freedom in designating radiographic conditions is intensified.

An X-ray CT apparatus in accordance with the ninth aspect of the present invention includes: an X-ray data acquisition means for rotating an X-ray generator and an X-ray area detector, which has a matrix structure and is opposed to the X-ray generator, about a center of rotation located between the X-ray generator and X-ray area detector, and for acquiring X-ray projection data of a subject lying down near the center of rotation; an image reconstruction means for treating the X-ray projection data acquired by the X-ray data acquisition means so as to reconstruct an image; an image display means for displaying a reconstructed tomographic image; and a radiographic condition designation means for use in designating radiographic conditions for data acquisition. The radiographic condition designation means is used to designate a plurality of radiographic conditions for a conventional scan for which the X-ray generator and X-ray area detector make one rotation or a cine scan for which the position of the subject is held unchanged in a z direction substantially orthogonal to a rotation plane on which the rotation is made. The image reconstruction means performs image reconstruction under the plurality of sets of radiographic conditions during the conventional scan or cine scan.

According to the ninth aspect of the present invention, the radiographic condition designation means is used to designate a plurality of sets of radiographic conditions for a conventional scan for which the X-ray generator and X-ray area detector make one rotation or a cine scan for which the position of the subject is fixed in a depth direction substantially orthogonal to the plane on which the rotation is made. The image reconstruction means performs image reconstruction under the plurality of sets of radiographic conditions during the conventional scan or cine scan. Thus, the radiographic conditions are optimized and freedom in designating the radiographic conditions is intensified.

An X-ray CT apparatus in accordance with the tenth aspect of the present invention includes: an X-ray data acquisition means for rotating an X-ray generator and an X-ray area detector, which has a matrix structure and is opposed to the X-ray generator, about a center of rotation located between the X-ray generator and X-ray area detector, and for acquiring X-ray projection data of a subject lying down near the center of rotation; an image reconstruction means for treating the X-ray projection data acquired by the X-ray data acquisition means so as to reconstruct an image; an image display means for displaying a reconstructed tomographic image; and a radiographic condition designation means for use in designating radiographic conditions for data acquisition. The X-ray data acquisition means irradiates an X-ray beam, which is asymmetric in a direction orthogonal to the rotation plane, to the rotation plane on which the rotation is made, so as to acquire X-ray data. The radiographic condition designation means is used to designate a plurality of sets of radiographic conditions for a conventional scan for which the X-ray generator and X-ray area detector make one rotation or a cine scan for which the position of the subject is fixed in a depth direction substantially orthogonal to the rotation plane. The image reconstruction means performs image reconstruction under the plurality of sets of radiographic conditions during the conventional scan or cine scan.

According to the tenth aspect of the present invention, the X-ray data acquisition means irradiates an X-ray beam, which is asymmetric in a direction orthogonal to the rotation plane, to the rotation plane on which the rotation is made. The radiographic condition designation means is used to designate a plurality of sets of radiographic conditions for a conventional scan for which the X-ray generator and X-ray area detector make one rotation or a cine scan for which the position of the subject is fixed in a depth direction substantially orthogonal to the rotation plane. The image reconstruction means performs image reconstruction under a plurality of sets of radiographic conditions during the conventional scan or cine scan. Thus, in relation to the asymmetric X-ray beam, the radiographic conditions are optimized and freedom in designating the radiographic conditions is intensified.

An X-ray CT apparatus in accordance with the eleventh aspect of the present invention is identical to the X-ray CT apparatus in accordance with the tenth aspect except that the X-ray data acquisition means includes a collimator having an asymmetric structure and being disposed in front of an X-irradiation port of the X-ray generator.

According to the eleventh aspect of the present invention, even when the X-ray collimator is asymmetric in a z direction, since the radiographic condition designation means for use in designating a plurality of sets of radiographic conditions for a conventional scan for which the X-ray generator and X-ray area detector make one rotation or a cine scan of scanning one position in the z direction, and the image reconstruction means are included, the radiographic conditions can be optimized and freedom in designating the radiographic conditions can be intensified.

An X-ray CT apparatus in accordance with the twelfth aspect of the present invention includes: an X-ray data acquisition means for rotating an X-ray generator and an X-ray area detector, which has a matrix structure and is opposed to the X-ray generator, about a center of rotation located between the X-ray generator and X-ray area detector, and for acquiring X-ray projection data of a subject lying down near the center of rotation; an image reconstruction means for treating the X-ray projection data acquired by the X-ray data acquisition means so as to reconstruct an image; an image display means for displaying a reconstructed tomographic image; and a radiographic condition designation means for use in designating radiographic conditions for data acquisition. The X-ray data acquisition means acquires data with a rotation plane, on which the rotation is made, tilted. The radiographic condition designation means is used to designate a plurality of sets of radiographic conditions for a conventional scan for which the X-ray generator and X-ray area detector make one rotation or a cine scan for which the position of the subject is fixed in a depth direction substantially orthogonal to the rotation plane on which the rotation is made. The image reconstruction means performs image reconstruction under the plurality of sets of radiographic conditions during the conventional scan or cine scan.

According to the twelfth aspect of the present invention, the X-ray data acquisition means acquires data with the rotation plane, on which the rotation is made, tilted. The radiographic condition designation means is used to designate a plurality of sets of radiographic conditions for a conventional scan for which the X-ray generator and X-ray area detector make one rotation or a cine scan for which the position of the subject is fixed in a depth direction substantially orthogonal to the rotation plane on which the rotation is made. The image reconstruction means performs image reconstruction under the plurality of sets of radiographic conditions during the conventional scan or cine scan. Thus, with the rotation plane tilted, the radiographic conditions are optimized and freedom in designating the radiographic conditions is intensified.

An X-ray CT apparatus in accordance with the thirteenth aspect of the present invention includes: an X-ray data acquisition means for rotating an X-ray generator and an X-ray area detector, which has a matrix structure and is opposed to the X-ray generator, about a center of rotation located between the X-ray generator and X-ray area detector, and for acquiring X-ray projection data of a subject lying down near the center of rotation; an image reconstruction means for treating the X-ray projection data acquired by the X-ray data acquisition means so as to reconstruct an image; an image display means for displaying a reconstructed tomographic image; a radiographic condition designation means for use in designating radiographic conditions for data acquisition; and a radiographic condition storage means in which radiographic conditions recommended for each radiographic region of the subject are stored. For a conventional scan for which the X-ray generator and X-ray area detector make one rotation or a cine scan for which the position of the subject is fixed in a depth direction substantially orthogonal to the plane on which the rotation is made, the radiographic condition designation means is used to delineate a radiographic region in a scout image of the subject, retrieves radiographic conditions recommended for the delineated radiographic region, and designates a plurality of radiographic conditions according to the recommended radiographic conditions. The image reconstruction means performs image reconstruction under the plurality of sets of radiographic conditions during the conventional scan or cine scan.

According to the thirteenth aspect of the present invention, for a conventional scan for which the X-ray generator and X-ray area detector make one rotation or a cine scan for which the position of the subject is fixed in a depth direction substantially orthogonal to the plane on which the rotation is made, the radiographic condition designation means is used to delineate a radiographic region in a scout image of the subject, retrieves radiographic conditions recommended for the delineated radiographic region, and designates a plurality of sets of radiographic conditions according to the recommended radiographic conditions. The image reconstruction means performs image reconstruction under the plurality of sets of radiographic conditions during the conventional scan or cine scan. Since a radiographic region is delineated in a scout image of a subject, the radiographic conditions recommended for each radiographic region are automatically designated.

An X-ray CT apparatus in accordance with the fourteenth aspect of the present invention is identical to the X-ray CT apparatus in accordance with any of the ninth to thirteenth aspects except that the X-ray area detector is a multi-array X-ray detector or a flat-panel X-ray detector.

According to the fourteenth aspect of the present invention, the two-dimensional X-ray area detector may be either a multi-array detector or a flat-panel X-ray detector. Whether an X-ray beam is asymmetric or symmetric in a z direction, since the radiographic condition designation means to be used to designate a plurality of sets of radiographic conditions for a conventional (axial) scan for which the X-ray generator and X-ray area detector make one rotation or a cine scan of scanning one position in the z direction, and the image reconstruction means are included, the radiographic conditions can be optimized and freedom in designating the radiographic conditions can be intensified.

An X-ray CT apparatus in accordance with the fifteenth aspect of the present invention is identical to the X-ray CT apparatus in accordance with any of the ninth to fourteenth aspects except that the radiographic conditions include at least one of a slice thickness, a scan field, a reconstruction function, an image filter, a tomographic-image tilt angle, a position of a tomographic image, and an inter-tomographic image spacing.

According to the fifteenth aspect of the present invention, if the radiographic condition designation means can be used to designate a slice thickness, a scan field, an image filter, a tomographic-image tilt angle, and a position of a tomographic image, the radiographic conditions can be optimized or the radiographic conditions can be designated freely.

An X-ray CT apparatus in accordance with the sixteenth aspect of the present invention is identical to the X-ray CT apparatus in accordance with any of the ninth to fifteenth aspects except that the image reconstruction means includes a three-dimensional image reconstruction means.

According to the sixteenth aspect of the present invention, a z filter is used to freely vary a slice thickness, and a tomographic-image tilt angle and a position of a tomographic image can be freely varied. Thus, radiographic conditions are optimized and freedom in designating the radiographic conditions is intensified.

According to an X-ray CT apparatus or an X-ray CT image reconstruction method in which the present invention is implemented, radiographic conditions for a conventional scan for which an X-ray generator and an X-ray detector make one rotation or a cine scan for which the scanned position of a subject is fixed can be optimized or the radiographic conditions can be designated freely, though freedom in designating the radiographic conditions in relation to one rotational irradiation of an X-ray cone beam has been limited in the past. When a plurality of organs or any other objects of radiography are scanned with one rotational irradiation of an X-ray cone beam, the radiographic conditions can be optimized relative to each organ or each object of radiography.

The invention will now be described in greater detail, by way of example, with reference to the drawings, in which:-

Fig. 1 is a block diagram showing an X-ray CT apparatus in accordance with an embodiment of the present invention.

Fig. 2 is an explanatory diagram showing the rotation of an X-ray generator (X-ray tube) and a multi-array X-ray detector.

Fig. 3 is a flowchart outlining actions to be performed in the X-ray CT apparatus in accordance with the embodiment of the present invention.

Fig. 4 is a flowchart describing preprocessing.

Fig. 5 is a flowchart describing three-dimensional image reconstruction.

Fig. 6 includes conceptual diagrams showing projection of lines on a field of view in a direction of X-ray transmission.

Fig. 7 is a conceptual diagram showing lines projected on the surface of a detector.

Fig. 8 is a conceptual diagram showing projection of X-ray projection data items Dr(view,x,y) onto the field of view.

Fig. 9 is a conceptual diagram showing back projection pixel data items D2 of respective pixels constituting the field of view.

Fig. 10 is an explanatory diagram showing production of back X-ray projection data items D3 through pixel-by-pixel addition of sets of back projection pixel data items D2 derived from all view.

Fig. 11 includes conceptual diagrams showing projection of lines on a circular field of view in the direction of X-ray transmission.

Fig. 12 shows a plurality of radiographic conditions (protocols) to be designated in relation to an X-ray cone beam (in a case where a slice thickness is varied).

Fig. 13 shows a plurality of radiographic conditions (protocols) to be designated in relation to an X-ray cone beam (in a case wherein a tomographic-image tilt angle is varied).

Fig. 14 shows a plurality of radiographic conditions (protocols) to be designated in relation to an X-ray cone beam (in a case where a scanner gantry is tilted).

Fig. 15 shows a plurality of radiographic conditions (protocols) to be designated in relation to an X-ray cone beam using a different beam formation X-ray filter (in a case where various radiographic conditions are varied).

Fig. 16 shows a plurality of radiographic conditions (protocols) to be designated in relation to an X-ray cone beam (in a case where respective regions are designated).

Fig. 17 shows a plurality of radiographic conditions (protocols) to be designated in relation to an X-ray cone beam that is asymmetric in a z direction.

Fig. 18 is a flowchart describing a conventional scan or a cine scan for which a plurality of radiographic conditions can be designated.

Fig. 19 shows examples of a scan type selection screen image and a scan parameter display screen image respectively.

Fig. 20 shows conventional radiographic condition designation.

Fig. 21 shows radiographic conditions (protocols) to be designated in relation to a plurality of organs.

Fig. 22 shows X-ray CT using a conventional asymmetric X-ray beam.

Fig. 23 shows an example of tomography using an asymmetric X-ray beam.

Referring to the drawings, the best mode for implementing an X-ray CT method and an X-ray CT apparatus in accordance with the present invention will be described below. Noted is that the present invention will not be limited to the best mode.

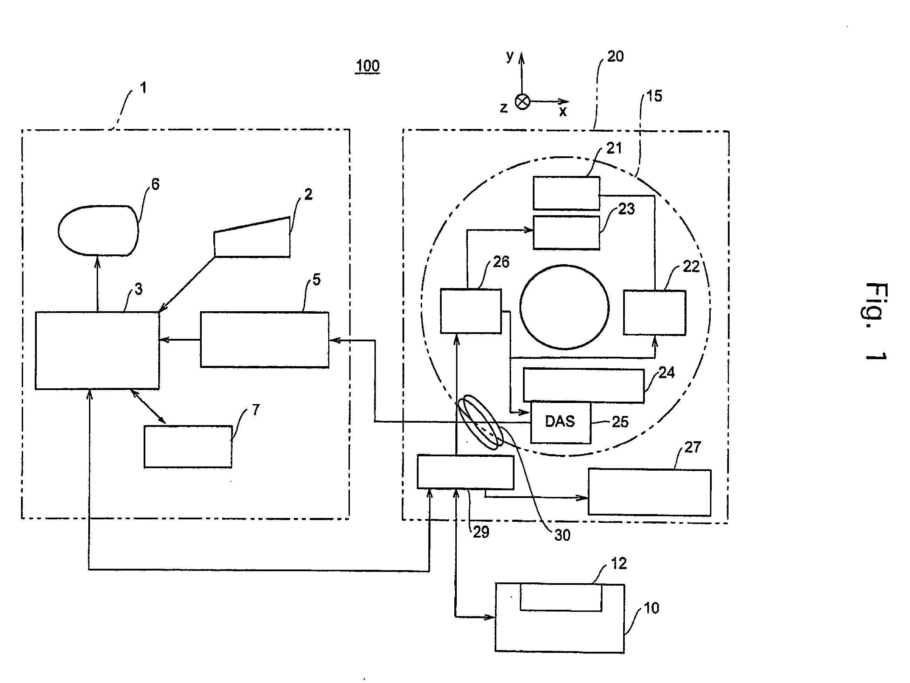

Fig. 1 is a block diagram showing the overall configuration of an X-ray CT apparatus in accordance with an embodiment of the present invention. The X-ray CT apparatus 100 includes an operator console 1, a radiographic table 10, and a scanner gantry 20.

The operator console 1 includes an input device 2 that receives an operator's input, a central processing unit 3 that includes an image reconstruction means for executing preprocessing, image reconstruction, and post-processing, a data collection buffer 5 into which X-ray detector data acquired by the scanner gantry 20 is collected, a monitor 6 that is an image display means on which a tomographic image reconstructed based on X-ray projection data produced by preprocessing the X-ray detector data is displayed, and a storage device 7 in which programs, X-ray detector data, X-ray projection data, and tomographic images are stored. Herein, the input device 2 and monitor 6 constitute a radiographic condition designation means for use in designating radiographic conditions as described later. The storage device 7 has the capability of a radiographic condition storage means in which designated radiographic conditions and radiographic conditions recommended for each radiographic region are stored. The radiographic conditions will be described later.

The radiographic table 10 includes a cradle 12 that carries a subject, who lied down on the cradle 12, into or out of the bore of the scanner gantry 20. The cradle 12 is raised, lowered, or rectilinearly moved by means of a motor incorporated in the radiographic table 10.

The scanner gantry 20 includes a control unit 29 that transfers control signals or the like to or from the operator console 1 and radiographic table 10, and a rotator 15. The rotator 15 includes an X-ray tube 21 serving as an X-ray generator, an X-ray controller 22, a collimator 23, a multi-array X-ray detector 24 that is an X-ray area detector, a data acquisition system (DAS) 25, and a rotator controller 26 that controls the X-ray tube 21 which rotates about the body axis of a subject. The rotator 15 is electrically connected to the scanner gantry 20 via a slip ring 3. The slip ring 30 serves as an annular electric contact arranged on the periphery of the rotator 15, permits non-contact data transfer through optical communication or electrostatic coupling, and enables the rotator 15 to continuously rotate in one direction. Herein, the rotator 15 serves as an X-ray data acquisition means for acquiring X-ray projection data of a subject.

Moreover, the scanner gantry 20 can be tilted forward or backward at approximately $\pm$ 30° with respect to a vertical direction by means of a gantry tilt controller 27. In the drawing, x, y, and z coordinate axes are fixed coordinate axes and are commonly applied to all drawings. The γ-axis direction corresponds to the vertical direction, and the x-axis and γ-axis directions are horizontal directions. The z-axis direction corresponds to a depth direction in which a subject lying down on the cradle 12 is moved in the bore. When the scanner gantry is not tilted, the xy plane corresponds to a rotation plane on which the X-ray tube 21 and multi-array X-ray detector 24 are rotated.

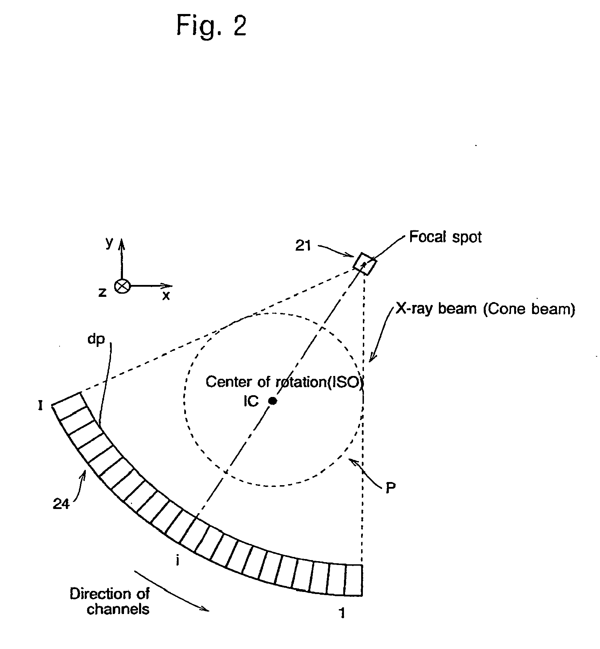

Fig. 2 is an explanatory diagram showing the geometric disposition of the X-ray tube 21 that is an X-ray generator and the multi-array X-ray detector 24 that is an X-ray area detector. The X-ray tube 21 and multi-array X-ray detector 24 are rotated about a center of rotation IC. Herein, the depth direction in which the cradle 12 is moved corresponds to the z direction all the time. The rotation plane on which the X-ray tube 21 and multi-array detector 24 are rotated corresponds to the xy plane as long as the scanner gantry is not tilted.

The X-ray tube 21 generates an X-ray beam called a cone beam CB. When the direction of the center axis of the cone beam CB is parallel to the y direction, it shall be said that the X-ray tube 21 is set to a view angle 0°.

The multi-array X-ray detector 24 includes, for example, 256 detector arrays. Moreover, each of the detector arrays includes, for example, 1024 detector channels juxtaposed in a direction of channels.

X-ray projection data acquired after irradiation of X-rays is detected by the multi-array X-ray detector 24, analog-to-digital converted by the DAS 25, and then transferred to the data collection buffer 5 via the slip ring 30. The data transferred to the data collection buffer 5 is treated by the central processing unit 3 according to a program read from the storage device 7, and a reconstructed tomographic image is displayed on the monitor 6.

The components of the X-ray CT apparatus in accordance with the present embodiment have been briefly described so far. In the X-ray CT apparatus 100 having the components, the rotator 15 serving as an X-ray data acquisition means acquires X-ray projection data as described below.

To begin with, a subject is carried into the bore of the rotator 15 of the scanner gantry 20. The position in the z-axis direction of the subject is fixed, an X-ray beam is irradiated from the X-ray tube 21 to the subject (projection of X-rays), and transmitted X-rays are detected by the multi-array X-ray detector 24. The detection of transmitted X-rays is performed with the X-ray tube 21 and multi-array X-ray detector 24 rotated about the subject (namely, with a view angle, that is, an angle of projection varied). In other words, data is acquired by rotating the X-ray tube 21 360° about a subject or by setting the X-ray tube 21 to N view angles (for example, N equals 1000).

The transmitted X-rays that have been detected are converted into digital data by the DAS 25, and transferred as X-ray projection data to the operator console 1 via the data collection buffer 5. This sequence of actions may be called a scan. The scanned position is sequentially shifted in the z-axis direction by a predetermined length, whereby subsequent scans are performed. This scanning method is referred to as a conventional or axial scanning method. On the other hand, a method in which projection data is acquired while the radiographic table 10 is moved at a predetermined speed synchronously with a change in a projection angle in order to thus shift a scanned position, that is, while the X-ray tube 21 and multi-array X-ray detector 24 are helically rotated about the subject is referred to as a helical scanning method. A method in which the position of the subject is fixed in the z direction without a shift of a scanned position in the z-axis direction, and one scan is repeatedly performed in order to acquire a time-sequential change in a tomographic image of the same radiographic region is referred to as a cine scanning method. A method in which the X-ray tube 21 and multi-array X-ray detector 24 are held at fixed positions but are not rotated, a scanned position is shifted in the z-axis direction by a predetermined length, and a projection image to be reconstructed by projecting X-rays on a subject in one direction is acquired is referred to as a scout scanning method.

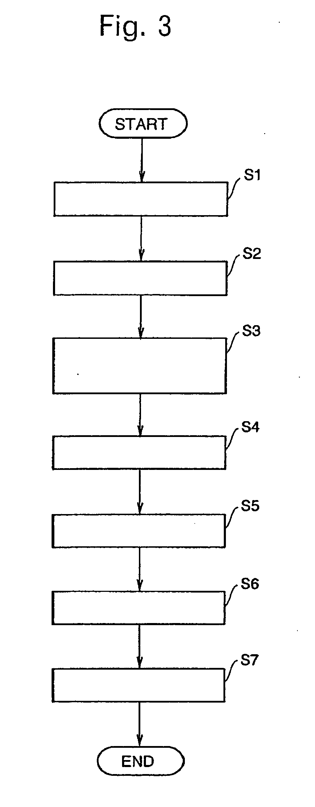

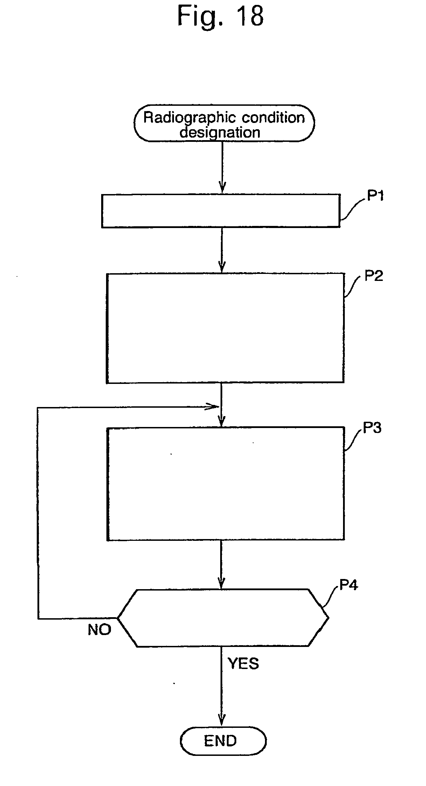

Actions to be performed in the X-ray CT apparatus 100 of the present invention will be outlined below. Fig. 18 is a flowchart describing actions to be performed during radiographic condition designation during which a plurality of radiographic conditions for a conventional scan or a cine scan is designated. Fig. 3 outlines actions to be performed during the conventional scan or cine scan executed under the radiographic conditions.

Fig. 18 is a flowchart describing radiographic condition designation.

At step P1, an operator executes a scout scan so as to acquire scout image data. Scout image data may be acquired in a plurality of directions including a direction of 0° (y-axis direction) and a direction of 90° (x-axis direction).

At step P2, the operator console 1 is used to delineate a region, of which data will be acquired during a conventional scan for which the X-ray tube and X-ray detector make one rotation or a cine scan of scanning one position in a z direction, in a scout image. A width determined with some detector arrays and the number of detector arrays are designated in advance, whereby a range in the z direction within which data is acquired is determined.

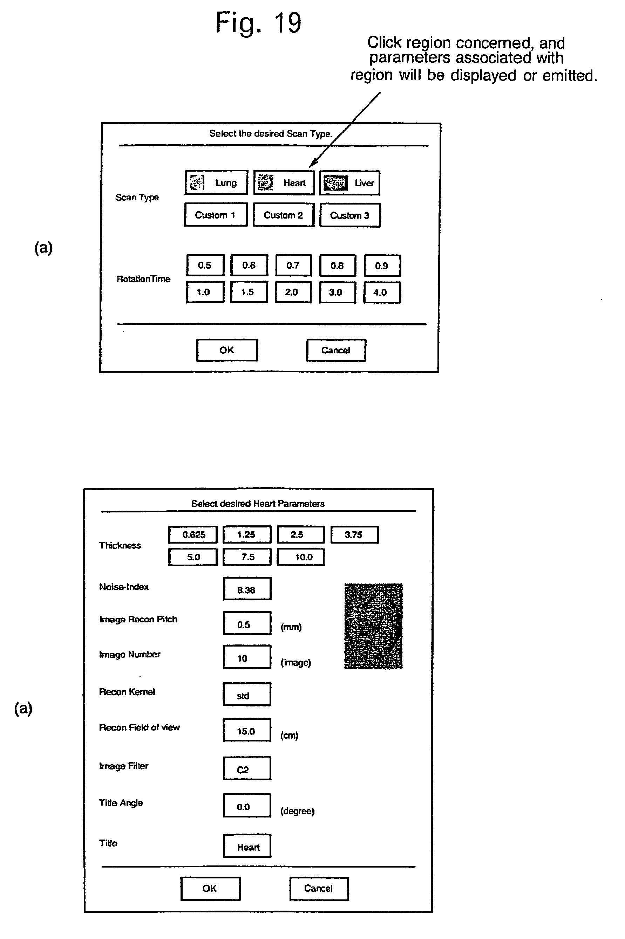

At step P3, the operator designates radiographic conditions for scanning of part of the region displayed at step P2. The operator may select radiographic conditions, which are recommended for a radiographic region and registered in advance in the memory, through a scan type selection screen image. Fig. 19 shows an example of the scan type selection screen image displayed on the monitor 6. Fig. 19(a) shows an example of the scan type selection image screen image allowing an operator to select radiographic conditions associated with each radiographic region. Fig. 19(b) shows an example of radiographic conditions recommended for the radiographic region selected through the scan type selection screen image from among sets of radiographic conditions registered in advance in association with respective radiographic regions.

At step P4, the operator repeats radiographic condition designation until he/she has designated a plurality of sets of radiographic conditions for a conventional scan or a cine scan. In this case, the radiographic conditions include radiographic conditions or parameters listed below.

    1. Slice thickness

    2. Scan field

    3. Reconstruction function

    4. Image filter

    5. Tomographic-image tilt angle

    6. Position of a tomographic image and inter-tomographic image spacing

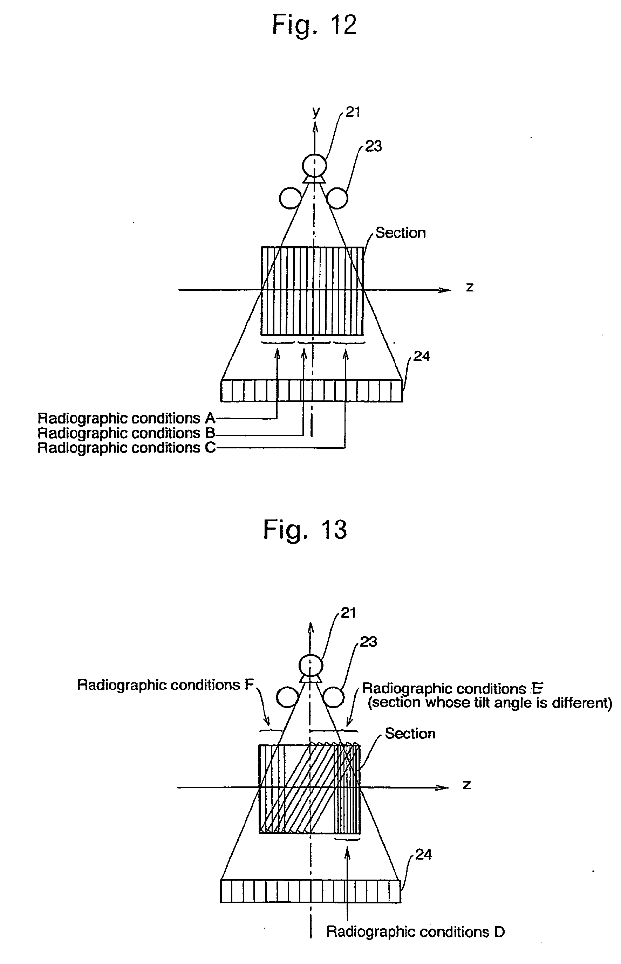

Fig. 12 shows a case where a slice thickness that is one of a plurality of radiographic conditions (protocols) to be designated in relation to an X-ray cone beam is varied. Fig. 12 shows a yz section of the rotator 15, that is, shows the scanner gantry 20 seen from a lateral direction that is an x direction. Moreover, a plurality of sections of a subject whose positions in the z direction are different from one another and which are imaged during one scan are illustrated near the center of rotation about which the X-ray tube 21 and multi-array X-ray detector 24 are rotated. Herein, the operator designates different sets of radiographic conditions A to C in association with the respective sections. Tomographic images of the sections associated with the different sets of radiographic conditions are produced during one scan.

Fig. 13 shows a case where a slice thickness and a tomographic-image tilt angle included in a plurality of radiographic conditions (protocols) to be designated in relation to an X-ray cone beam are varied. Fig. 13 shows, similarly to Fig. 12, the scanner gantry 20 seen from a lateral direction, that is, the x direction. In Fig. 13, the tomographic-image tilt angle included in the radiographic conditions E is different from the tomographic-image tilt angle that is included in each of the radiographic conditions D and radiographic conditions F and that indicates right sections oriented in a vertical direction. A tilt angle of sections whose tomographic images are produced during one scan is different among the sets D to F of radiographic conditions.

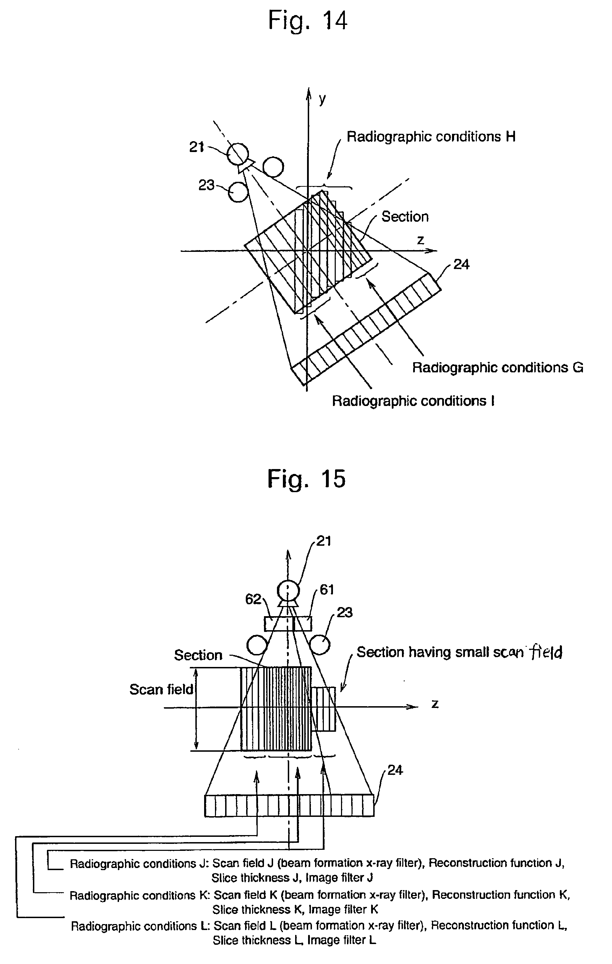

Fig. 14 shows a case where a slice thickness included in a plurality of radiographic conditions (protocols) to be designated in relation to an X-ray cone beam with the scanner gantry 20 tilted is varied. Fig. 14 shows, similarly to Fig. 12, the scanner gantry 20 seen from a lateral direction that is the x direction. In Fig. 14, with the scanner gantry 20 tilted, a tomographic-image tilt angle included in the radiographic conditions H indicates a vertical direction, and the tomographic-image tilt angle included in the radiographic conditions G or I indicates the same direction as the direction determined with the tilt angle of the scanner gantry 20. The sets G to I of radiographic conditions are different from one another in terms of a slice thickness.

Fig. 15 shows a case where some radiographic conditions included in a plurality of radiographic conditions (protocols) to be designated in relation to an X-ray cone beam are varied. Fig. 15 shows, similarly to Fig. 12, the scanner gantry 20 seen from a lateral direction that is the x direction. Referring to Fig. 15, different beam formation X-ray filters (wedge filters) 61 and 62 that control the intensity of an X-ray beam on an xy plane are included. A scan field on the xy plane to be imaged during one scan is varied depending on a position in the z-axis direction. Herein, radiographic conditions J stipulate a small scan field J, while radiographic conditions K or L stipulates a standard-size scan field K or L. Incidentally, the sets J, K, and L of radiographic conditions are different from one another in terms of a reconstruction function, a slice thickness, and an image filter.

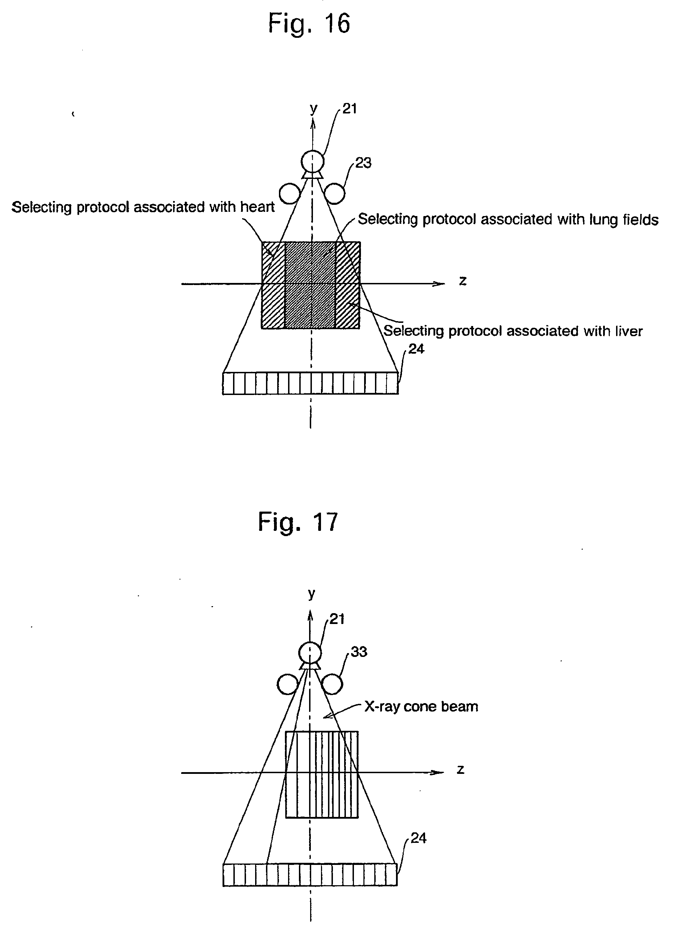

Fig. 16 shows a case where a plurality of sets of recommended radiographic conditions that is registered in advance is automatically designated as a plurality of sets of radiographic conditions (protocols) in relation to an X-ray cone beam merely by designating a radiographic region. Fig. 16 shows, similarly to Fig. 12, the scanner gantry 20 seen in a lateral direction that is the x direction. Referring to Fig. 16, different sets of radiographic conditions are designated in association with respective radiographic regions through the scan type selection screen image like the one shown in Fig. 19.

Fig. 17 shows a case where an X-ray beam that is asymmetric in the z direction is irradiated, and a plurality of sets of radiographic conditions is designated as a plurality of sets of radiographic conditions (protocols) to be designated in relation to an X-ray cone beam. Fig. 17 shows, similarly to Fig. 12, the scanner gantry 20 seen from a lateral direction that is the x direction. Referring to Fig. 17, the collimator 33 is disposed asymmetrically to the xy plane in order to reshape an X-ray cone beam, which is irradiated from the X-ray tube 21, asymmetrically in the z-axis direction. A plurality of sets of radiographic conditions is designated according to the shape of the X-ray cone beam asymmetric in the z-axis direction.

Next, scanning actions to be performed after completion of radiographic condition designation will be outlined below.

At step S1 in Fig. 3, the X-ray tube 21 and multi-array X-ray detector 24 are rotated about a subject in order to perform a conventional scan or a cine scan. X-ray projection data D0(view,j,i) identified with a view angle view, a detector array number j, and a channel number i is acquired with a position ztable(view) in a z direction of rectilinear table movement appended thereto.

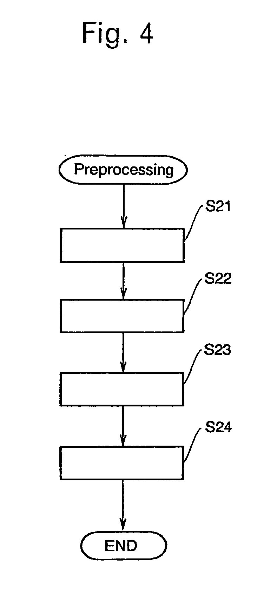

At step S2, the image reconstruction means included in the central processing unit 3 performs preprocessing on X-ray detector data D0(view,j,i) or converts it into X-ray projection data. Fig. 4 is a flowchart describing actions to be performed for preprocessing. During the preprocessing, offset nulling is performed (step S21), logarithmic conversion is performed (step S22), X-ray dose correction is performed (step S23), and sensitivity correction is performed (step S24).

At step S3, the image reconstruction means performs beam hardening correction on the preprocessed X-ray projection data D1(view,j,i). During beam hardening correction S3, assuming that X-ray projection data having undergone sensitivity correction S23 included in the preprocessing S2 is Din and data having undergone beam hardening correction S3 is Dout, the beam hardening correction S3 is expressed with, for example, a polynomial as follows:

[Formula 1]

$$Dout(view,j,i) =$$

$$Din(view,j,i) \cdot (B_0(j,i) + B_1(j,i) \cdot Din(view,j,i) + B_2(j,i) \cdot Din(view,j,i)^2)$$

Since beam hardening correction can be independently performed on data detected by each detector array j, if a tube voltage for a data acquisition system that is one of radiographic conditions is varied depending on the detector array, a difference of an X-ray energy characteristic of each detector array can be corrected.

At step S4, the image reconstruction means performs z filter convolution so as to filter the preprocessed X-ray projection data D0(view,j,i) in the z direction (direction of arrays).

At step S4, a filter whose size in the direction of arrays is five arrays and which is expressed below is applied to X-ray projection data items which are acquired by respective data acquisition systems at each view angle and detected by respective detector elements Det(ch,row) (where ch denotes 1 to CH and row denotes 1 to ROW) of the multi-array detector (see Fig. 12).

$(w_1(ch), w_2(ch), w_3(ch), w_4(ch), w_5(ch))$

[Formula 2]

$$\sum_{k=1}^{5} W_k(ch) = 1$$

Corrected detector data Dcor(ch,row) is expressed as follows:

[Formula 3]

$$Dcor(ch, j) \sum_{k=1}^{5} (\det(ch, i - k - 3) \cdot W_k(ch))$$

Assuming that CH denotes a maximum channel number and ROW denotes a maximum detector array number, the following formula is defined:

[Formula 4]

Det(ch,-1) = Det(ch,0) = Det(ch,1)

Det(ch,ROW) = Det(ch,ROW+1) = Det(ch,ROW+2)

When array-direction filtering coefficients are varied depending on each channel, a slice thickness can be controlled according to a distance from the center of reconstruction. In general, the slice thickness of a tomographic image is larger in the perimeter of the tomographic image than in the center of reconstruction thereof. Therefore, the array-direction filtering coefficients are differentiated between the center of a tomographic image and the perimeter thereof. Specifically, when the filter is applied to data items detected on respective channels near the center of the detector, the variance of the array-direction filtering coefficients is widened. When the filter is applied to data items detected on respective channels near the edge of the detector, the variance of the array-direction filtering coefficients is narrowed. In this case, the slice thickness of a tomographic image becomes equal between the perimeter of the tomographic image and the center of reconstruction thereof.

Since the array-direction filtering coefficients to be applied to data items detected on respective channels near the center of the multi-array X-ray detector 24 or data items detected on respective channels near the edge thereof are controlled as mentioned above, the slice thickness can be controlled differently between the center of a tomographic image and the perimeter thereof. When the slice thickness is increased using the array-direction filter, both artifacts and noises are largely reduced. Consequently, the degree of reducing artifacts or noises can be controlled. Eventually, the quality of a tomographic image resulting from three-dimensional image reconstruction or the image quality realized on the xy plane can be controlled. In other embodiment, the array-direction (z-direction) filtering coefficients are determined to realize a de-convolution filter so that a tomographic image of a small slice thickness can be constructed.

At step S5, the image reconstruction means performs reconstruction function convolution. Namely, image data is Fourier-transformed, applied a reconstruction function, and then inverse-Fourier-transformed. During the reconstruction function convolution S5, assuming that data having undergone z filter convolution is Din, data having undergone reconstruction function convolution is Dout, a reconstruction function to be convoluted is Kernel(j), and convolution is *, the reconstruction function convolution is expressed as follows:

## [Formula 5]

$$Dout(view,j,i) = Din(view,j,i) * Kernel(j)$$

Since the reconstruction function Kernel(j) can be convoluted to data detected by each j array of the detector, the difference of the noise or resolution characteristic of data detected by each detector array can be corrected.

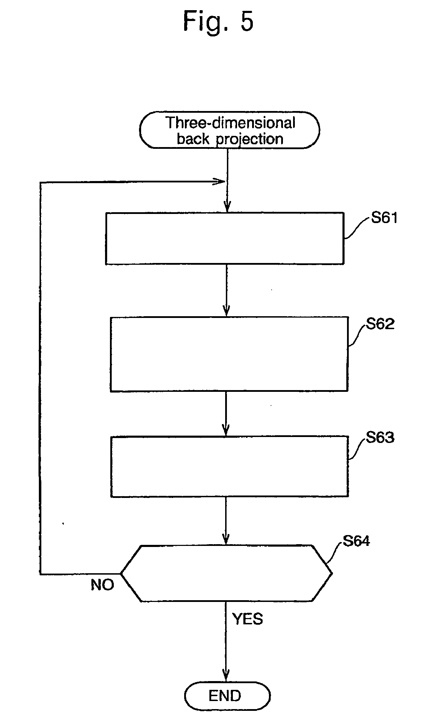

At step S6, the image reconstruction means performs three-dimensional back projection on X-ray projection data D0(view,j,i) having undergone reconstruction function convolution so as to produce back X-ray projection data D3 (x,y). Although the present invention adopts the helical scanning method, an image to be three-dimensionally reconstructed relative to a plane perpendicular to the z axis, that is, the xy plane. A field of view P shall be parallel to the xy plane. The three-dimensional back projection will be described later with reference to Fig. 5.

At step S7, the image reconstruction means performs post-processing including image filter convolution and CT number transform on the back X-ray projection data D3(x,y) so as to reconstruct a tomographic image. During image filter convolution included in post-processing, assuming that data having undergone three-dimensional back projection is Din(x,y,z), data having undergone image filter convolution is Dout(x,y,z), an image filter is Filter(z), and convolution is *, the following formula is defined:

## [Formula 6]

$$Dout(x,y,z) = Din(x,y,z) * Filter(z)$$

Since the image filter can be independently convoluted to data detected by each j array of the detector, the difference of the noise or resolution characteristic of data detected by each array can be corrected. A resultant tomographic image is displayed on the monitor 6.

Fig. 5 is a flowchart describing three-dimensional back projection (step S6 in Fig. 3). Although the present invention adopts the helical scanning method, an image is three-dimensionally reconstructed relative to a plane perpendicular to the z axis, that is, the xy plane. A field of view P shall be parallel to the xy plane.

At step S61, the image reconstruction means samples X-ray projection data items Dr, which are associated with respective pixels constituting the field of view P, from one of all views required to reconstruct a tomographic image (that is, views detected with the X-ray tube rotated 360° or 180° + the angle of a fan-shaped beam).

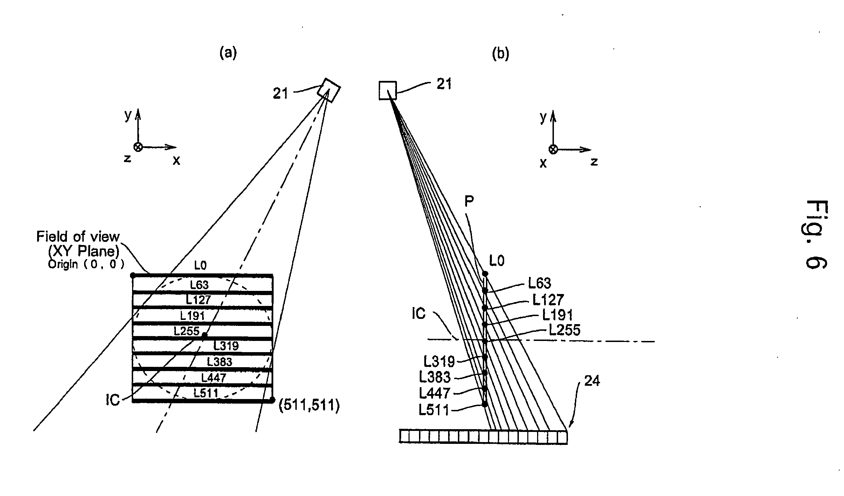

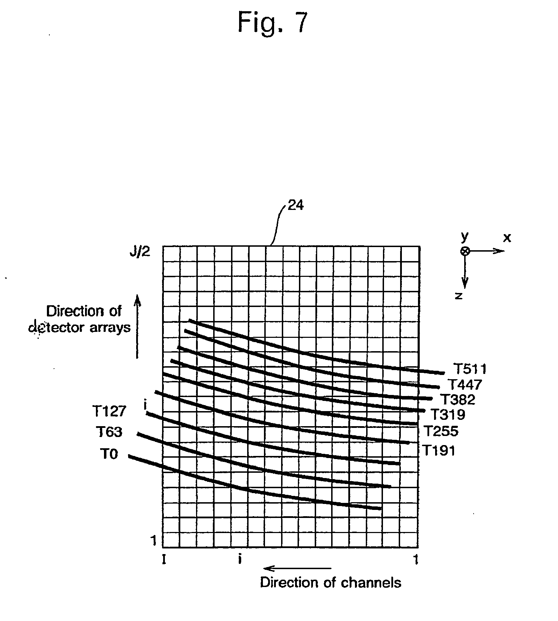

As shown in Fig. 6(a) and Fig. 6(b), the field of view P is a square field that has 512 pixels lined in rows and columns and that is parallel to the xy plane. Assume that a line of pixels L0 parallel to the x axis at a position of y=0, a line of pixels L63 at a position of y=63, a line of pixels L127 at a position of y=127, a line of pixels L191 at a position of y=191, a line of pixels L255 at a position of y=255, a line of pixels L319 at a position of y=319, a line of pixels L383 at a position of y=383, a line of pixels L447 at a position of y=447, and a line of pixels L511 at a position of y=511 are projected on the surface of the multi-array X-ray detector 24 in a direction of X-ray transmission. X-ray projection data items forming lines T0 to T511 like the ones shown in Fig. 7 are then sampled and regarded as sets of X-ray projection data items Dr associated with the lines of pixels L0 to L511.

The direction of X-ray transmission depends on the geometric positions of the focal spot on the X-ray tube 21, each pixel, and the multi-array X-ray detector 24. Since the z-coordinate z(view) representing the location of X-ray detector data D0(view,j,i) is appended as data of a positional in a z direction of rectilinear table movement, Ztable (view), to the X-ray detector data, the direction of X-ray transmission can be accurately sampled from X-ray projection data D0 (view,j,i) irrespectively of whether the X-ray projection data D0(view,j,i) is detected during acceleration or deceleration of the scanner gantry.

When a line partly comes out of the multi-array X-ray detector 24 in the direction of channels in the same manner as a line T0 that is formed on the surface of the multi-array X-ray detector 24 by projecting the line of pixels L0 in the direction of X-ray transmission does, associated X-ray projection data items Dr are set to 0s. When a line partly comes out of the multi-array X-ray detector 24 in the z direction, missing X-ray projection data items Dr are extrapolated.

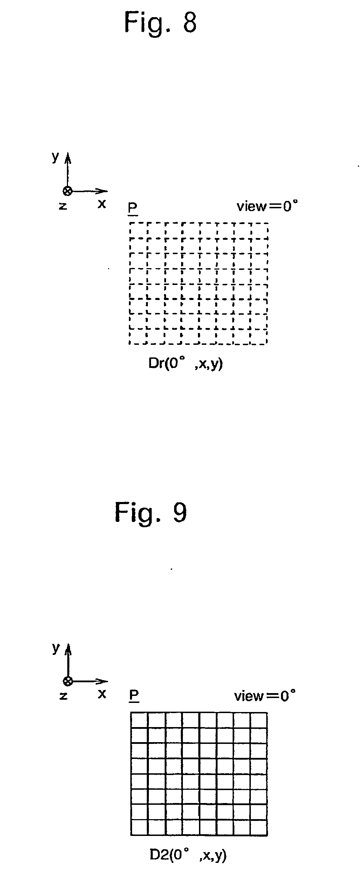

Consequently, as shown in Fig. 8, X-ray projection data items Dr(view,x,y) associated with pixels constituting the field of view P are sampled.

Referring back to Fig. 5, at step S62, the image reconstruction means multiplies X-ray projection data items Dr(view,x,y) by a cone-beam reconstruction weighting coefficient so as to produce X-ray projection data items D2(view, x,y) like the ones shown in Fig. 9. Herein, the cone-beam reconstruction weighting coefficient w(i,j) is expressed below. In case of fan-beam image reconstruction, assuming that an angle at which a straight line linking the focal spot in the X-ray tube 21 and a pixel g(x,y) on the field of view P (xy plane) meets the center axis Bc of an X-ray beam irradiated at a view angle view=βa is γ, and an opposite view angle is view=βb, the view angle βb is expressed as follows:

$$\beta b = \beta a + 180° - 2\gamma$$

Assuming that angles at which the X-ray beam passing through the pixel g(x,y) on the field of view P and the opposite X-ray beam meet the field of view P are αa and αb respectively, X-ray projection data items are multiplied by cone-beam reconstruction weighting coefficients ωa and ωb dependent on the angles. The resultant sets of data items are summated in order to produce back projection pixel data items D2(0,x,y).

$$D2(0,x,y) = \omega a \cdot D2(0,x,y)\_a + \omega b \cdot D2(0,x,y)\_b$$

Incidentally, the sum of the cone-beam reconstruction weighting coefficients associated with the opposed X-ray beams comes to unity.

$$\omega a + \omega b = 1$$

Since the sets of data items are multiplied by the cone-beam reconstruction weighting coefficients ωa and ωb and then summated, conical-angle artifacts can be minimized. For example, the cone-beam reconstruction weighting coefficients ωa and ωb may be calculated according to formulae presented below. Assume that a half of the angle of a fan beam is γmax.

[Formula 7]

$$ga = f(\gamma max, \alpha a, \beta a)$$

$$gb = f(\gamma max, \alpha b, \beta b)$$

$$xa = 2 \cdot ga^q/(ga^q + gb^q)$$

$$xb = 2 \cdot gb^q/(ga^q + gb^q)$$

$$\omega a = xa^2 \cdot (3 \cdot 2xa)$$

$$\omega b = xb^2 \cdot (3 \cdot 2xb)$$

where q equals, for example, 1.

For example, assuming that ga and gb are functions providing max[A,B] where a larger one of A and B is adopted, ga and gb are rewritten as follows:

[Formula 8]

$$ga = \max[0,\{(\pi/2 + \gamma\max)\cdot \ |\beta a|\}] \cdot \ |\tan(\alpha a)|$$

$$gb = \max[0,\{(\pi/2 + \gamma\max)\cdot \ |\beta b|\}] \cdot \ |\tan(\alpha b)|$$

In the case of fan-beam image reconstruction, pixels on the field of view P are multiplied by a distance coefficient. The distance coefficient is provided as $(r1/r0)^2$ where a distance from the focal spot in the X-ray tube 21 to a detector element of the multi-array X-ray detector 24 that belongs to a detector array j and a channel i and that detects X-ray projection data Dr is r0 and a distance from the focal spot in the X-ray tube 21 to a pixel on the field of view P associated with the X-ray projection data Dr is r1.

In the case of parallel-ray beam image reconstruction, the respective pixels on the field of view P are multiplied by the cone-beam reconstruction weighting coefficient w(i,j) alone.

At step S63, the image reconstruction means adds pixel by pixel, as shown in Fig. 10, X-ray projection data items D2(view,x,y) to back X-ray projection data items D3(x,y) that are cleared.

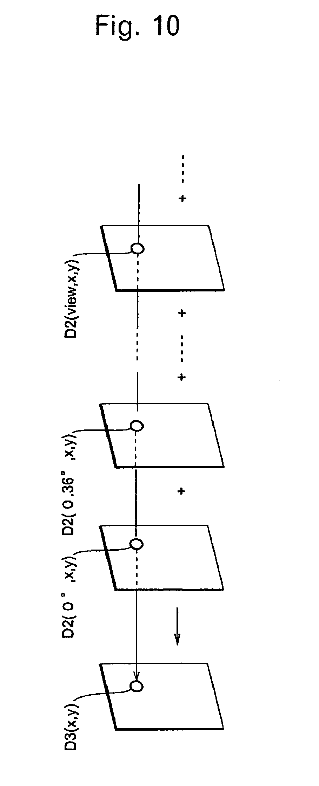

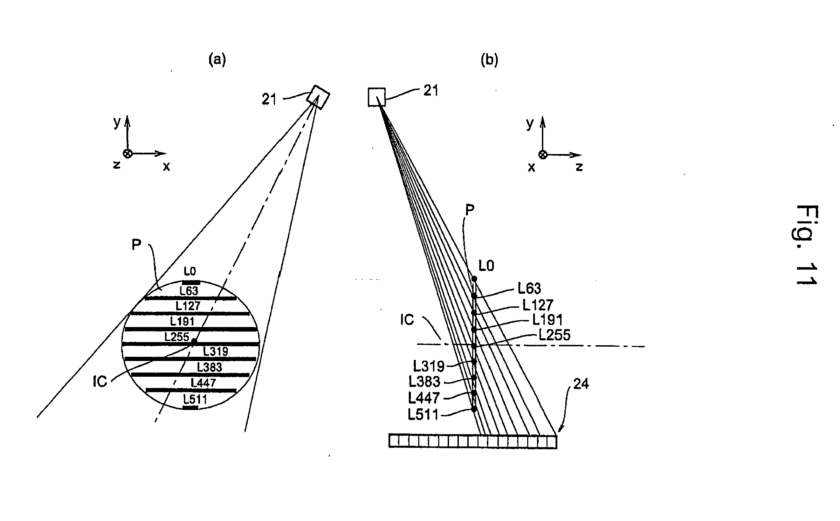

At step S64, the image reconstruction means repeats steps S61 to S63 for all views required to reconstruct a tomographic image, that is, views detected with the X-ray tube rotated 360° (or 180° + the angle of a fan-shaped beam) so as to produce back X-ray projection data items D3(x,y) as shown in Fig. 10. As shown in Fig. 11 (a) and Fig. 11 (b), the field of view P may be circular.

According to the X-ray CT method in accordance with the present invention implemented in the X-ray CT apparatus 100, or according to the X-ray CT apparatus 100, although a conventional X-ray CT apparatus including the multi-array X-ray detector 24 has little freedom in designating radiographic conditions for a conventional scan for which an X-ray generator and an X-ray area detector make one rotation or a cine scan, which scans one position in the z direction, in relation to one rotational irradiation of an X-ray cone beam, and cannot optimize the radiographic conditions, the radiographic conditions can be optimized or designated freely. Moreover, when a plurality of organs or any other objects of radiography are scanned with one rotational irradiation of an X-ray cone beam, the radiographic conditions can be optimized relative to each organ or each object of radiography.

According to the present embodiment, the multi-array X-ray detector 24 is adopted as an X-ray area detector. Alternatively, a flat-panel X-ray detector may be substituted for the multi-array X-ray detector 24.

According to the present embodiment, a three-dimensional image reconstruction method based on a known Feldkamp technique may be adopted as an image reconstruction method. Otherwise, any other three-dimensional image reconstruction method may be adopted.

The present embodiment has been described on the assumption that the X-ray CT apparatus 100 for medical use is adopted. Alternatively, an X-ray CT apparatus for industrial use may be adopted or an X-ray CT apparatus may be used in combination with any other system such as an X-ray CT-PET system or an X-ray CT-SPECT apparatus.

**Claims**

**1.** An X-ray CT method comprising the steps of:

rotating an X-ray generator (21) and an X-ray area detector (24), which has a matrix structure and is opposed to the X-ray generator (21), about a center of rotation located between the X-ray generator (21) and X-ray area detector (24) so as to acquire X-ray projection data of a subject lying down near the center of rotation; reconstructing an image by treating the acquired X-ray projection data; displaying the reconstructed tomographic image; and designating radiographic conditions for data acquisition, wherein:

a plurality of sets of radiographic conditions is designated for a conventional scan for which the X-ray generator (21) and X-ray area detector (24) make one rotation or a cine scan for which the position of the subject is held unchanged in a depth direction substantially orthogonal to a rotation plane on which the rotation is made; and

an image is reconstructed under the plurality of sets of radiographic conditions during the conventional scan or cine scan.

**2.** An X-ray CT method comprising the steps of:

rotating an X-ray generator (21) and an X-ray area detector (24), which has a matrix structure and is opposed to the X-ray generator (21), about a center of rotation located between the X-ray generator (21) and X-ray area detector (24) so as to acquire X-ray projection data of a subject lying down near the center of rotation; reconstructing an image by treating the acquired X-ray projection data; displaying the reconstructed tomographic image; and designating radiographic conditions for data acquisition, wherein:

for data acquisition, an X-ray beam that is asymmetric to a direction orthogonal to a rotation plane on which the rotation is made is irradiated to the rotation plane; a plurality of sets of radiographic conditions is designated for a conventional scan for which the X-ray generator (21) and X-ray area detector (24) make one rotation or a cine scan for which the position of the subject is fixed in a depth direction substantially orthogonal to the rotation plane on which the rotation is made; and an image is reconstructed under the plurality of sets of radiographic conditions during the conventional scan or cine scan.

**3.** An X-ray CT method comprising the steps of:

rotating an X-ray generator (21) and an X-ray area detector (24), which has a matrix structure and is opposed to the X-ray generator (21), about a center of rotation located between the X-ray generator (21) and X-ray area detector (24) so as to acquire X-ray projection data of a subject lying down near the center of rotation; reconstructing an image by treating the acquired X-ray projection data; displaying the reconstructed tomographic image; and designating radiographic conditions for data acquisition, wherein:

prior to data acquisition, a rotation plane on which the rotation is made is tilted; a plurality of sets of radiographic conditions is designated for a conventional scan for which the X-ray generator (21) and X-ray area detector (24) make one rotation or a cine scan for which the position of the subject is fixed in a depth direction substantially orthogonal to the rotation plane on which the rotation is made; and an image is reconstructed under the plurality of sets of radiographic conditions during the conventional scan or cine scan.

**4.** An X-ray CT method comprising the steps of:

rotating an X-ray generator (21) and an X-ray area detector (24), which has a matrix structure and is opposed to the X-ray generator (21), about a center of rotation located between the X-ray generator (21) and X-ray area detector (24) so as to acquire X-ray projection data of a subject lying down near the center of rotation; reconstructing an image by treating the acquired X-ray projection data; displaying the reconstructed tomographic image; designating radiographic conditions for data acquisition; and storing radiographic conditions recommended for each radiographic region of the subject, wherein:

for a conventional scan for which the X-ray generator (21) and X-ray area detector (24) make one rotation or a cine scan for which the position of the subject is fixed in a depth direction substantially orthogonal to a rotation plane on which the rotation is made, a radiographic region is delineated in a scout image of the subject, radiographic conditions recommended for the delineated radiographic region are retrieved, and a plurality of sets of radiographic conditions is designated based on the recommended radiographic conditions; and an image is reconstructed under the plurality of sets of radiographic conditions during the conventional scan or cine scan.

5. The X-ray CT method according to any of Claims 1 to 4, wherein the radiographic conditions include at least one of a slice thickness, a scan field, a reconstruction function, an image filter, a tomographic-image tilt angle, a position of a tomographic image, and an inter-tomographic image spacing.

6. An X-ray CT apparatus (100) comprising:

an X-ray data acquisition device (20, 5) for rotating an X-ray generator (21) and an X-ray area detector (24), which has a matrix structure and is opposed to the X-ray generator (21), about a center of rotation located between the X-ray generator (21) and X-ray area detector (24) so as to acquire X-ray projection data of a subject lying down near the center of rotation;
an image reconstruction device (3) for reconstructing an image by treating the X-ray projection data acquired by the X-ray data acquisition device (20, 5);
an image display device (6) for displaying the reconstructed tomographic image; and
a radiographic condition designation device (2) for use in designating radiographic conditions for data acquisition, wherein:

the radiographic condition designation device (2) is used to designate a plurality of sets of radiographic conditions for a conventional scan for which the X-ray generator (21) and X-ray area detector (24) make one rotation or a cine scan for which the position of the subject is held unchanged in a z direction substantially orthogonal to a rotation plane on which the rotation is made; and
the image reconstruction device (3) reconstructs an image under the plurality of sets of radiographic conditions during the conventional scan or cine scan.

7. An X-ray CT apparatus (100) comprising:

an X-ray data acquisition device (20, 5) for rotating an X-ray generator (21) and an X-ray area detector (24), which has a matrix structure and is opposed to the X-ray generator (21), about a center of rotation located between the X-ray generator (21) and X-ray area detector (24) so as to acquire X-ray projection data of a subject lying down near the center of rotation;
an image reconstruction device (3) for reconstructing an image by treating the X-ray projection data acquired by the X-ray data acquisition device (20, 5);
an image display device (6) for displaying the reconstructed tomographic image; and
a radiographic condition designation device (2) for use in designating radiographic conditions for data acquisition, wherein:

the X-ray data acquisition device (20, 5) irradiates an X-ray beam, which is asymmetric to a direction orthogonal to a rotation plane on which the rotation is made, to the rotation plane so as to acquire X-ray data;
the radiographic condition designation device (2) is used to designate a plurality of sets of radiographic conditions for a conventional scan for which the X-ray generator (21) and X-ray area detector (24) make one rotation or a cine scan for which the position of the subject is fixed in a depth direction substantially orthogonal to the rotation plane on which the rotation is made; and
the image reconstruction device (3) reconstructs an image under the plurality of sets of radiographic conditions during the conventional scan or cine scan.

8. An X-ray CT apparatus (100) comprising:

an X-ray data acquisition device (20, 5) for rotating an X-ray generator (21) and an X-ray area detector (24), which has a matrix structure and is opposed to the X-ray generator (21), about a center of rotation located between the X-ray generator (21) and X-ray area detector (24) so as to acquire X-ray projection
an image reconstruction device (3) for reconstructing an image by treating the X-ray projection data acquired by the X-ray data acquisition device (20, 5);
an image display device (6) for displaying the reconstructed tomographic image; and
a radiographic condition designation device (2) for use in designating radiographic conditions for data acquisition, wherein:

the X-ray data acquisition device (20, 5) acquires data with a rotation plane, in which the rotation is made, tilted;
the radiographic condition designation device (2) is used to designate a plurality of sets of radiographic conditions for a conventional scan for which the X-ray generator (21) and X-ray area detector (24) make one rotation or a cine scan for which the position of the subject is fixed in a depth direction substantially orthogonal to the rotation plane on which the rotation is made; and

the image reconstruction device (3) reconstructs an image under the plurality of sets of radiographic conditions during the conventional scan or cine scan.

9. An X-ray CT apparatus (100) comprising:

an X-ray data acquisition device (20, 5) for rotating an X-ray generator (21) and an X-ray area detector (24), which has a matrix structure and is opposed to the X-ray generator (21), about a center of rotation located between the X-ray generator (21) and X-ray area detector (24) so as to acquire X-ray projection data of a subject lying down near the center of rotation;

an image reconstruction device (3) for reconstructing an image by treating the X-ray projection data acquired by the X-ray data acquisition device (20, 5);

an image display device (6) for displaying the reconstructed tomographic image;

a radiographic condition designation device (2) for use in designating radiographic conditions for data acquisition; and

a radiographic condition storage device (7) in which radiographic conditions recommended for each radiographic region of the subject are stored, wherein:

for a conventional scan for which the X-ray generator (21) and X-ray area detector (24) make one rotation or a cine scan for which the position of the subject is fixed in a depth direction substantially orthogonal to a rotation plane on which the rotation is made, the radiographic condition designation device (2) is used to delineate a radiographic region in a scout image of the subject, retrieve radiographic conditions recommended for the delineated radiographic region, and designate a plurality of sets of radiographic conditions according to the recommended radiographic conditions; and

the image reconstruction device (3) reconstructs an image under the plurality of sets of radiographic conditions during the conventional scan or cine scan.

10. The X-ray CT apparatus (100) according to any of Claims 6 to 9, wherein the radiographic conditions include at least one of a slice thickness, a scan field, a reconstruction function, an image filter, a tomographic-image tilt angle, a position of a tomographic image, and an inter-tomographic image spacing.

# Fig. 1

# Fig. 2

# Fig. 3

```
        ┌─────────────┐
        │    START    │
        └──────┬──────┘
               │
               ▼
    ┌──────────────────────┐  S1
    │                      │
    └──────────┬───────────┘
               │
               ▼
    ┌──────────────────────┐  S2
    │                      │
    └──────────┬───────────┘
               │
               ▼
    ┌──────────────────────┐  S3
    │                      │
    │                      │
    └──────────┬───────────┘
               │
               ▼
    ┌──────────────────────┐  S4
    │                      │
    └──────────┬───────────┘
               │
               ▼
    ┌──────────────────────┐  S5
    │                      │
    └──────────┬───────────┘
               │
               ▼
    ┌──────────────────────┐  S6
    │                      │
    └──────────┬───────────┘
               │
               ▼
    ┌──────────────────────┐  S7
    │                      │
    └──────────┬───────────┘
               │
               ▼
        ┌─────────────┐
        │     END     │
        └─────────────┘
```

# Fig. 4

# Fig. 5

# Fig. 6

# Fig. 7

# Fig. 8

y

z  x  P                    view＝0°

Dr(0° ,x,y)

# Fig. 9

y

z  x  P                    view＝0°

D2(0° ,x,y)

# Fig. 10

# Fig. 11

EP 1 736 102 A2

Fig. 12

Radiographic conditions A
Radiographic conditions B
Radiographic conditions C

Fig. 13

Radiographic conditions F

Radiographic conditions E
(section whose tilt angle is different)

Section

Radiographic conditions D

28

## Fig. 14

21
23
y
Radiographic conditions H
Section
z
24
Radiographic conditions G
Radiographic conditions I

## Fig. 15

21
62
61
23
Section
Section having small scan field
Scan field
z
24

Radiographic conditions J: Scan field J (beam formation x-ray filter), Reconstruction function J,
Slice thickness J, Image filter J
Radiographic conditions K: Scan field K (beam formation x-ray filter), Reconstruction function K,
Slice thickness K, Image filter K
Radiographic conditions L: Scan field L (beam formation x-ray filter), Reconstruction function L,
Slice thickness L, Image filter L

# Fig. 16

Selecting protocol associated with heart

Selecting protocol associated with lung fields

Selecting protocol associated with liver

# Fig. 17

X-ray cone beam

# Fig. 18

```
        ┌─────────────────────────────┐
        │  Radiographic condition     │
        │        designation          │
        └─────────────────────────────┘
                      │
                      ▼
        ┌─────────────────────────────┐  P1
        │                             │╱
        └─────────────────────────────┘
                      │
                      ▼
        ┌─────────────────────────────┐  P2
        │                             │╱
        │                             │
        │                             │
        └─────────────────────────────┘
    ┌──────────────────►│
    │                   ▼
    │   ┌─────────────────────────────┐  P3
    │   │                             │╱
    │   │                             │
    │   │                             │
    │   └─────────────────────────────┘
    │                   │
    │                   ▼
    │        ╱───────────────────╲  P4
    └───────<                     >╱
       NO    ╲───────────────────╱
                      │
                     YES
                      │
                      ▼
               ┌───────────┐
               │   END     │
               └───────────┘
```

# Fig. 19

Click region concerned, and
parameters associated with
region will be displayed or emitted.

**(a)**

Select the desired Scan Type.

Scan Type

| Lung | Heart | Liver |
|------|-------|-------|
| Custom 1 | Custom 2 | Custom 3 |

RotationTime

| 0.5 | 0.6 | 0.7 | 0.8 | 0.9 |
|-----|-----|-----|-----|-----|
| 1.0 | 1.5 | 2.0 | 3.0 | 4.0 |

| OK | Cancel |

**(a)**

Select desired Heart Parameters

Thickness

| 0.625 | 1.25 | 2.5 | 3.75 |
|-------|------|-----|------|
| 5.0 | 7.5 | 10.0 | |

Noise-Index    8.38

Image Recon Pitch    0.5    (mm)

Image Number    10    (image)

Recon Kernel    std

Recon Field of view    15.0    (cm)

Image Filter    C2

Title Angle    0.0    (degree)

Title    Heart

| OK | Cancel |

## Fig. 20

Section

z

One set of radiographic conditions

## Fig. 21

Example of
radiographic conditions

Heart : 1mm thick

Lung : 10mm thick

Liver : 2.5mm thick

Heart

Lung

Liver

z

For example, 0.625 mm by 512 arrays

## Fig. 22

## Fig. 23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10305027 A